(19) Europäisches Patentamt
European Patent Office
Office européen des brevets

(11) Publication number: **0 095 451**
**A2**

(12) # EUROPEAN PATENT APPLICATION

(21) Application number: **83850127.8**

(22) Date of filing: **13.05.83**

(51) Int. Cl.³: **C 07 D 211/90**
**A 61 K 31/445**

(30) Priority: **21.05.82 SE 8203176**

(43) Date of publication of application:
**30.11.83 Bulletin 83/48**

(84) Designated Contracting States:
**AT BE CH DE FR GB IT LI LU NL SE**

(71) Applicant: **Aktiebolaget Hässle**
**Kärragatan 5**
**S-431 83 Mölndal(SE)**

(72) Inventor: **Berntsson, Peder Bernhard**
**Flugsnapparegatan 17A**
**S-431 33 Mölndal(SE)**

(72) Inventor: **Carlsson, Stig Ake Ingemar**
**Vallmovägen 3**
**S-435 00 Mölnlycke(SE)**

(72) Inventor: **Ljung, Bengt Richard**
**Torild Wulffsgatan 34**
**S-413 19 Göteborg(SE)**

(74) Representative: **Wurm, Bengt Runio et al,**
**Patent and Trade Mark Department AB ASTRA**
**S-151 85 Södertälje(SE)**

(54) **Novel dihydropyridines.**

(57) New compounds useful as therapeutical agent and useful as intermediates for the preparation of other therapeutically active dihydropyridines, which new compounds are of the formula

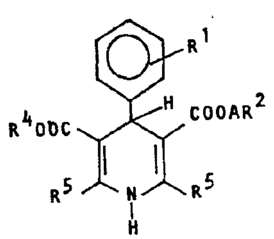

wherein $R^1$ is one or two (the same or different) substituents on the phenyl ring selected from the group consisting of lower alkyl, $NO_2$, CN, $CF_3$, Cl and Br; A is a straight or branched alkylene group with 2-5 carbon atoms; $R^2$ is

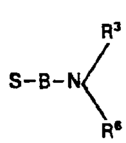

wherein B is a straight or branched alkylene group with 2-5 carbon atoms and $R^3$ and $R^6$ are the same or different and each represents a straight or branched alkyl group with 1-5 carbon atoms, an alkylaryl group or both together are part of a 5-7 membered heterocyclic ring; $R^4$ is a straight or branched alkyl group with 1-5 carbon atoms in which alkyl group the carbon chain is interrupted by an oxygen atom; $R^5$ is a lower alkyl group, or a physiologically acceptable salt or optical isomer thereof, processes for the preparation of said compounds; pharmaceutical preparations containing said compounds, a method for their therapeutical use and a new process using said compounds as intermediates.

EP 0 095 451 A2

# Novel dihydropyridines

## Description

### Field of the invention

The present invention relates to new compounds having valuable therapeutical properties, processes for their preparation, their use as intermediates for preparing other dihydropyridines, a new method for preparing such other dihydropyridines, a method for the treatment of cardiovascular diseases and other disorders where smooth muscle relaxation is therapeutically important in mammals including man and pharmaceutical preparations containing said compounds.

An object of the present invention is to provide new agents for the treatment of cardiovascular diseases and other disorders where smooth muscle relaxation is therapeutically important, particularly agents having antihypertensive properties.

Another object of the invention is to provide compounds useful as intermediates for the preparation of other dihydropyridines.

A further object of the invention is to provide a process for the preparation of such other dihydropyridines.

### Prior Art

Adalat® (nifedipine) having the formula

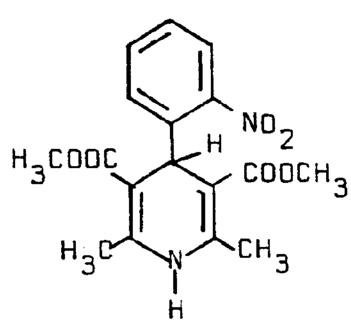

$R^3$ and $R^6$ are together part of a 5-7 membered heterocyclic ring is defined as for instance

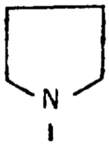 , 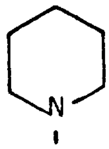 , 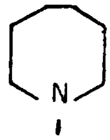

Straight or branched alkyl groups with 1-5 carbon atoms can be $-CH_3$, $-C_2H_5$, $-CH_2CH_2CH_3$, $-CH(CH_3)_2$, $-(CH_2)_3CH_3$, 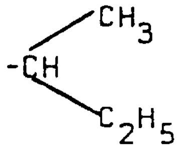 , $-CH_2CH(CH_3)_2$, $-C(CH_3)_3$, $-CH_2C(CH_3)_3$.

Straight or branched alkyl groups with 1-5 carbon atoms wherein the carbon chain is interrupted by an oxygen atom can be

$-CH_2CH_2OCH_3$, $-CH_2CH_2OC_2H_5$, 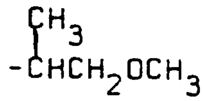, $-CH_2CH_2CH_2OCH_3$, 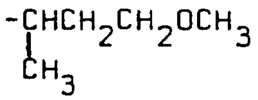.

Specific preferred compounds of the invention are

1) 2,6-dimethyl-4-(2,3-dichlorophenyl)-1,4-dihydropyridine--3,5-dicarboxylic acid-3-[2-(2-N,N-dimethylaminoethylthio)-ethyl]-5-methyl ester

2) 2,6-dimethyl-4-(2,3-dichlorophenyl)-1,4-dihydropyridine--3,5-dicarboxylic acid-3-[2-(2-N,N-dimethylaminoethylthio)-ethyl]-5-(2-methylethyl)ester

3) 2,6-dimethyl-4-(2,3-dichlorophenyl)-1,4-dihydropyridine--3,5-dicarboxylic acid-3-[2-(2-N,N-dimethylaminoethylthio)-ethyl]-5-ethyl ester

lower alkyl, $NO_2$, CN, $CF_3$, Cl and Br; A is a straight or branched alkylene group with 2-5 carbon atoms; $R^2$ is

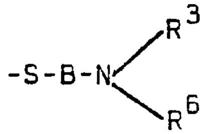

wherein B is a straight or branched alkylene group with 2-5 carbon atoms and $R^3$ and $R^6$ are the same or different and each represents a straight or branched alkyl group with 1-5 carbon atoms, an alkylaryl group or both together are part of a 5-7 membered heterocyclic ring; $R^4$ is a straight or branched alkyl group with 1-5 carbon atoms or a straight or branched alkyl group with 1-5 carbon atoms in which alkyl group the carbon chain is interrupted by an oxygen atom; $R^5$ is a lower alkyl group, or a physiologically acceptable salt or optical isomer thereof, possess a specific muscle relaxing effect.

Straight or branched alkylene groups with 2-5 carbon atoms can be $-CH_2CH_2-$, $-CH_2CH_2CH_2-$, $-CH_2CH_2CH_2CH_2-$, $-CH_2CH_2CH_2CH_2CH_2-$,

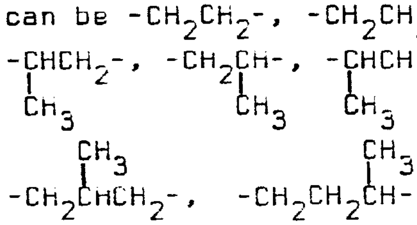

Lower alkyl is an alkyl group with 1-5 carbon atoms, e.g. methyl, ethyl, propyl, i-propyl, butyl, sec-butyl, i-butyl, tert-butyl.

Alkylaryl groups are $-CH_3-Ar$, $-CH(CH_3)-Ar$, $-(CH_2)_2-Ar$, $-CH(CH_3)CH_2-Ar$

wherein Ar is 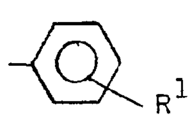

wherein $R^1$ is as defined above.

$R^3$ and $R^6$ are together part of a 5-7 membered heterocyclic ring is defined as for instance

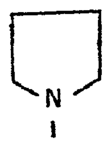 , 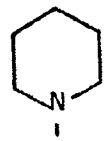 , 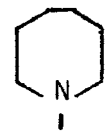

Straight or branched alkyl groups with 1-5 carbon atoms can be $-CH_3$, $-C_2H_5$, $-CH_2CH_2CH_3$, $-CH(CH_3)_2$, $-(CH_2)_3CH_3$,

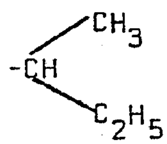 , $-CH_2CH(CH_3)_2$, $-C(CH_3)_3$, $-CH_2C(CH_3)_3$.

Straight or branched alkyl groups with 1-5 carbon atoms wherein the carbon chain is interrupted by an oxygen atom can be

$-CH_2CH_2OCH_3$, $-CH_2CH_2OC_2H_5$, 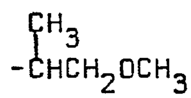, $-CH_2CH_2CH_2OCH_3$,

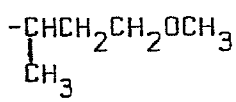.

Specific preferred compounds of the invention are

1) 2,6-dimethyl-4-(2,3-dichlorophenyl)-1,4-dihydropyridine-3,5-dicarboxylic acid-3-[2-(2-N,N-dimethylaminoethylthio)-ethyl]-5-methyl ester

2) 2,6-dimethyl-4-(2,3-dichlorophenyl)-1,4-dihydropyridine-3,5-dicarboxylic acid-3-[2-(2-N,N-dimethylaminoethylthio)-ethyl]-5-(2-methylethyl)ester

3) 2,6-dimethyl-4-(2,3-dichlorophenyl)-1,4-dihydropyridine-3,5-dicarboxylic acid-3-[2-(2-N,N-dimethylaminoethylthio)-ethyl]-5-ethyl ester

4) 2,6-dimethyl-4-(2,3-dichlorophenyl)-1,4-dihydropyridine-3,5-dicarboxylic acid-3-[2-(2-N,N-dimethylaminoetylthio-ethyl]-5-(2-methoxyethyl) ester

5) 2,6-dimethyl-4-(2,3-dichlorophenyl)-1,4-dihydropyridine-3,5-dicarboxylic acid-3-[2-(2-N,N-diethylaminoethylthio)-ethyl]-5-methyl ester

6) 2,6-dimethyl-4-(2,3-dichlorophenyl)-1,4-dihydropyridine-3,5-dicarboxylic acid-3-[2-(2-morpholinoethylthio)-ethyl]-5-methyl ester

7) 2,6-dimethyl-4-(2,3-dichlorophenyl)-1,4-dihydropyridine-3,5-dicarboxylic acid-3-[2-(N-isopropyl-N-benzylaminoethyl-thio)ethyl]-5-methyl ester

8) 2,6-dimethyl-4-(2,3-dichlorophenyl)-1,4-dihydropyridine-3,5-dicarboxylic acid-3-[2-(3-N,N-dimethylaminopropylthio)-ethyl]-5-methyl ester

9) 2,6-dimethyl-4-(2,3-dichlorophenyl)-1,4-dihydropyridine-3,5-dicarboxylic acid-3-[2-(2-N-isopropyl-N-benzylethyl-thio)ethyl]-5-(2-methoxyethyl) ester

10) 2,6-dimethyl-4-(2,3-dichlorophenyl)-1,4-dihydropyridine-3,5-dicarboxylic acid-3-[2-(2-morpholinoethylthio)-ethyl]-5-(2-methoxyethyl] ester

11) 2,6-dimethyl-4-(2-nitrophenyl)-1,4-dihydropyridine-3,5-dicarboxylic acid-3-[2-(2-N,N-dimethylaminoethylthio)ethyl]-5-methyl ester

12) 2,6-dimethyl-4-(3-nitrophenyl)-1,4-dihydropyridine-3,5--dicarboxylic acid-3-[2-(N,N-dimethylaminoethylthio)ethyl]--5-methyl ester

13) 2,6-dimethyl-4-(3-cyanophenyl)-1,4-dihydropyridine-3,5--dicarboxylic acid-3-[2-(2-N,N-dimethylaminoethylthio)ethyl]--5-methyl ester

The substances are intended to be administered orally or parenterally for acute and chronic treatment of above-mentioned cardiovascular disorders.

The new compounds are obtained according to methods known <u>per se</u>.

Thus,

$a^1$) a compound of the formula

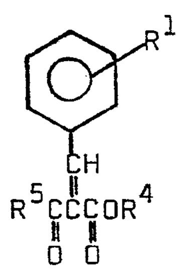

II a

wherein $R^1$, $R^4$ and $R^5$ have the meanings given above, is reacted with a compound of the formula

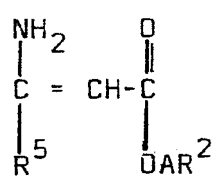

III a

wherein A and $R^2$ and $R^5$ have the meanings given above, to the formation of a compound of the formula I; or

$a^2$) a compound of the formula

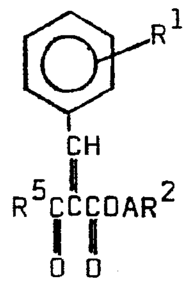

II b

wherein $R^1$, $R^2$, $R^5$ and A have the meanings given above, is reacted with a compound of the formula

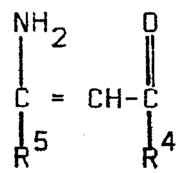

III b

wherein $R^4$ and $R^5$ have the meaning given above, to the formation of a compound of the formula I; or

$b^1$) a compound of the formula

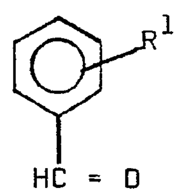

IV

wherein $R^1$ has the meaning given above, is reacted with compounds of the formulas

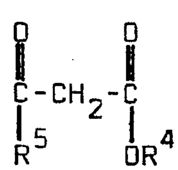

V a

and

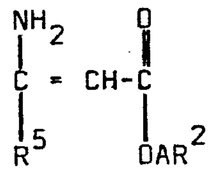

III a

wherein $R^2$, $R^4$, $R^5$ and A have the meanings given above, to the formation of a compound of the formula I; or

$b^2$) a compound of formula IV above, wherein $R^1$ has the meaning given above, is reacted with compounds of the formulas

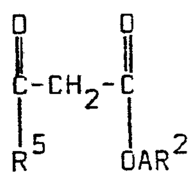

V b

and

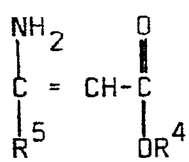

III b

wherein $R^2$, $R^4$, $R^5$ and A have the meanings given above, to the formation of a compound of the formula I; or

$c^1$) a compound of formula II a above, wherein $R^1$, $R^4$ and $R^5$ have the meanings given above, is reacted with a compound of the formula

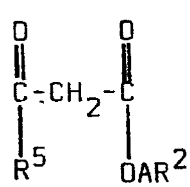

VI a

wherein $R^2$, $R^5$ and A have the meanings given above, in the presence of ammonia, to the formation of a compound of tbe formula I; or

$c^2$) a compound of formula II b above, wherein $R^1$, $R^2$, $R^5$ and A have tne meanings given above, is reacted with a compound of the formula

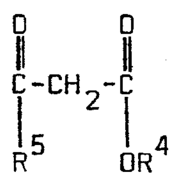

VI b

wherein $R^4$ and $R^5$ have the meanings given above, in the presence of ammonia, to the formation of a compound of the formula I, or

d) a compound of formula IV above, wherein $R^1$ has the meaning given above, is reacted with the compounds of the formulas V a and V b, wherein $R^2$, $R^4$, $R^5$ and A have the meanings given above, in the presence of ammonia, to the formation of a compound of the formula I, whereafter, if desired, the compound obtained by any of methods $a^1$) - d) is converted to a physiologically acceptable salt thereof and/or converted to a substantially pure stereoisomer thereof.

The reactions $a^1$) - d) are carried out in the presence or absence of a solvent, preferably in the presence of water or an organic solvent. The reactions are carried out at a temperature of 20-200$^0$C.

The invention also relates to any embodiment of the process of which one starts from any compound obtained as an intermediate in any process step and one carries out the lacking process step, or one breaks of the process at any step, or at which one forms a starting material under the reaction

conditions, or at which a reaction component possibly in the form of its salt is present.

The new compounds may, depending on the choice of starting materials and process, be present as optical antipodes or racemate, or, if they contain at least two asymmetric carbon atoms, be present as an isomer mixture (racemate mixture).

The isomer mixtures (racemate mixtures) obtained may, depending on physical-chemical differences of the components, be separated into the two stereoisomeric (diastereomeric) pure racemated e.g. by means of chromatography and/or fractional crystallization.

The racemate obtained can be separated according to known methods, e.g., by means of recrystallization from an optically active solvent,by means of microorganisms, by means of chromatography, or by a reaction with optically active acids forming salts of the compounds, and separating the salts thus obtained, e.g. by means of the different solubility of the diastereomeric salts, from which the antipodes may be set free by the action of a suitable agent. Suitably useable optically active acids are e.g. the L- and D-forms of tartaric acid, di-o-tolyltartaric acid, malic acid, mandelic acid, camphorsulfonic acid or quinic acid. Preferably the more active part of the two antipodes is isolated.

Suitably such starting materials are used for carrying out tbe reactions of the invention, which materials leads to groups of end products preferably desired and particularly to the specifically described and preferred end products.

The starting materials are known or may, if they are novel, be obtained according to processes known per se.

The present invention also pertains to the pharmaceutically acceptable nontoxic salts of these basic compounds. Such salts indludes those derived from organic and inorganic acids such as, without limitation, hydrochloric acid, hydrobromic acid, phosphoric acid, sulfuric acid, methane sulphonic acid, acetic acid, tartaric acid, lactic acid, succinic acid, citric acid, malic acid, maleic acid, sorbic acid, aconitic acid, salicyclic acid, phthalic acid, embonic acid, enanthic acid, or any acid that is acceptable as a food additive.

In clinical use the compounds of the invention are usually administered orally, or rectally in the form of a pharmaceutical preparation, which contains the active component as free base in combination with a pharmaceutically acceptable carrier.

Thus the mentioning of the new compounds of the invention is here related to the free amine base even if the compounds are generally or specifically described, provided that the context in which such expressions are used, e.g., in the examples, is not in disagreement with this meaning. The carrier may be a solid, semisolid or liquid diluent or a capsule. These pharmaceutical preparations are a further object of the invention.

Usually the amount of active compound is between 0.1 and 99% by weight of the preparation, suitably between 0.5 and 20% by weight in preparations for injection and between 2 and 50% by weight in preparations for oral administration.

In the preparation of pharmaceutical preparations containing a compound of the present invention in the form of dosage units for oral administration the compound elected may be mixed with a solid, pulverulent carrier, as e.g. with lactose, saccharose, sorbitol, mannitol, starch, such as potato starch, corn starch, amylopectin, cellulose derivatives or gelatine,

as well as with an antifriction agent such as magnesium stearate, calcium stearate, polyethyleneglycol waxes or the like, and be pressed into tablets. If coated tablets are wanted, the above prepared core may be coated with concentrated solution of sugar, which solution may contain, e.g., gum arabicum, gelatine, talc, titandioxide or the like. Furthermore, the tablets may be coated with a laquer dissolved in an easily volatile organic solvent or mixture of solvents. To this coating a dye may be added in order to easily distinguish between tablets with different active compounds or with different amounts of the active compound present.

In the preparation of soft gelatine capsules (pearl-shaped, closed capsules), which consist of gelatine and e.g., glycerine, or in the preparation of similar closed capsules, the active compound is mixed with a vegetable oil. Hard gelatine capsules may contain granules of the active compound in combination with a solid, pulverulent carrier as lactose, saccharose, sorbitol, mannitol, starch (as, e.g., potato starch, corn starch or amylopectin), cellulose derivatives or gelatine.

Dosage units for rectal administration may be prepared in the form of suppositories, which contain the active substance in a mixture with a neutral fat base, or they may be prepared in the form of gelatine-rectal capsules which contain the active substance in a mixture with a vegetable oil or paraffin oil.

Liquid preparations for oral administration may be present in the form of sirups or suspensions, e.g. solutions containing from about 0.2% by weight to about 20% by weight of the active substance described, glycerol and propylene glycol. If desired, such liquid preparations may contain

colouring agents, flavouring agents, saccharine and carboxymethylcellulose as a thickening agent.

The daily dose of the active substance varies and is dependent on the type of administration, but as a general rule it is 10 to 1000 mg/day of active substance at peroral administration.

## Intermediates

The compounds of formula I above are useful not only as therapeutically active agents but also as intermediates for the preparation of other dihydropyridines, such as compounds of the formula

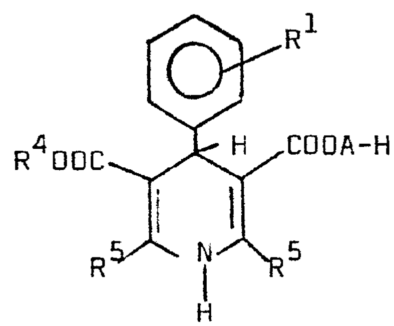

I A

wherein A is a straight or branched alkylene group with 2-5 carbon atoms and $R^1$ is one or two (the same or different) substituents on the phenyl ring selected from the group consisting of lower alkyl, $NO_2$, CN, $CF_3$, Cl or Br; $R^4$ is a straight or branched alkyl group with 1-5 carbon atoms or a straight or branched alkyl group with 1-5 carbon atoms in which alkyl group the carbon chain is interrupted by an oxygen atom; $R^5$ is a lower alkyl group; or a physiologically acceptable salt or optical isomer thereof.

Such compounds are previously known as antihypertensive agents. For instance, the compound 2,6-dimethyl-4-(2,3-dichlorophenyl)-1,4-dihydropyridine-3,5-dicarboxylic acid-3-ethyl-5-methyl ester (felodipine), which is disclosed in US patent No. 4 264 611.

New process

The generally employed processes for preparing a particular compound of the formula I A above results in the obtainment of a mixture of compounds with regard to the substituents on the ester groups. This mixture of symmetrical and slightly unsymmetrical substances is very difficult to separate into its various components, thus resulting in a highly unpure preparation.

According to the present invention this problem is solved by preparing an apparently highly unsymmetrical intermediate of formula I, which is an organic monofunctional base. By using its properties as an organic base it can be easily separated from undesired by-products and thereafter transferred to the pure substance of formula I A. The new process results in end products of a very high degree of purity; and especially with a low contamination of closely related dihydropyridine dicarboxylic acid esters.

The process is characterized in the treatment of a compound of the formula I above in a solvent with a Raney catalyst, whereby a compound of the formula I A above is obtained.

Suitable solvents are methanol, ethanol, dioxane, tetrahydrofurane, benzene, toluene, acetone, methylethylketone, ethylacetate.

As Raney catalysts can be used Raney nickel and Raney cobalt.

The process is suitable carried out at a temperature of 15-100$^{\circ}$C.

Best mode of carrying out the invention

The following illustrates the principles and the adaptation of the invention, however, without being limited thereto.

Example 1 (method $b^2$)

Preparation of 2,6-dimethyl-4-(2,3-dichlorophenyl)-1,4-dihydropyridine-3,5-dicarboxylic acid-3-[2-(2-N,N-dimethylaminoethylthio)ethyl]-5-methyl ester

A mixture of 2.9 g of 2,3-dichlorobenzaldehyde, 2.3 g of 3-aminocrotonic acid methyl ester, 4.7 g of aceto-acetic acid-2-(2-N,N-dimethylaminoethylthio)ethyl ester and 125 ml of isopropanol was refluxed over one night. The mixture was then evaporated and to the residue was added while stirring 100 ml of 0.4 M HCl. The mixture was extracted with ether. The water phase was then extracted with methylene chloride. The methylene chloride phase was then shaken with 1 M NaOH. After drying and evaporation of the methylene chloride the title compound was obtained as an oil $^{1}H$ and $^{13}C$-NMR spectra are consistent with the given structure. Yield: 37 %.

Example 2 (method $a^2$)

Preparation of 2,6-dimethyl-4-(2,3-dichlorophenyl)-1,4-dihydropyridine-3,5-dicarboxylic acid-3-[2-(2-morpholinoethylthio)ethyl]-5-(2-methoxyethyl) ester

A mixture of 12,9 g of 2,3-dichlorobenzylideneacetylacetic acid-2-(2-morpholinoethylthio)ethyl ester and 4,7 g of 3-aminocrotonic acid-2-methoxyethyl ester in 50 ml of pyridine was refluxed for 4 hours. The mixture was then evaporated and the residue dissolved in 100 ml of methylene chloride. The resulting solution was washed twice with 50 ml of 2 M HCl and then twice with 2 M NaOH. Drying and evaporation of the organic phase gave the title compound as an oil. $^{1}H$ and $^{13}C$ NMR spectra are consistent with the given structure. Yield: 61 %.

The following compounds have also been prepared (see Table I)

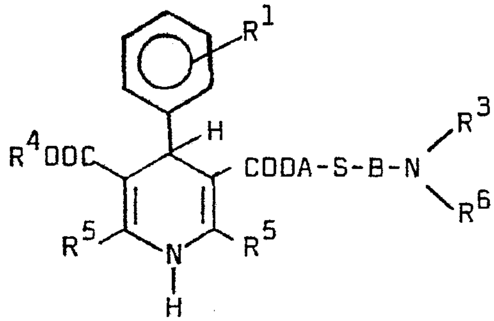

TABLE I

| Compound no. | $R^1$ | $R^5$ | $R^4$ | $R^3$ | $R^6$ | A | B | Method | Yield % | Mp. °C |
|---|---|---|---|---|---|---|---|---|---|---|
| 1 | 3-$NO_2$ | $CH_3$ | $CH_3$ | $CH_3$ | $CH_3$ | $(CH_2)_2$ | $(CH_2)_2$ | $b^2$ | 20 | 85-135 |
| 2 | 2-$NO_2$ | $CH_3$ | $CH_3$ | $CH_3$ | $CH_3$ | $(CH_2)_2$ | $(CH_2)_2$ | $b^2$ | 30 | 75 (HCl) |
| 3 | 3-CN | $CH_3$ | $CH_3$ | $CH_3$ | $CH_3$ | $(CH_2)_2$ | $(CH_2)_2$ | $b^2$ | 19 | 70 (HCl) |
| 4 | 2,3-di-Cl | $CH_3$ | $CH_3$ | $CH_3$ | $CH_3$ | $(CH_2)_2$ | $(CH_2)_2$ | $b^2$ | 36 | oil |
| 5 | " | $CH_3$ | $(CH_3)_2CH$ | $CH_3$ | $CH_3$ | $(CH_2)_2$ | $(CH_2)_2$ | $b^2$ | 6 | 80 (HCl) |
| 6 | " | $CH_3$ | $CH_3CH_2$ | $CH_3$ | $CH_3$ | $(CH_2)_2$ | $(CH_2)_2$ | $b^2$ | 10 | 90 (HCl) |
| 7 | " | $CH_3$ | $CH_3OCH_2CH_2$ | $CH_3$ | $CH_3$ | $(CH_2)_2$ | $(CH_2)_2$ | $b^2$ | 16 | 65-95 (HCl) |
| 8 | " | $CH_3$ | $CH_3$ | $CH_3CH_2$ | $CH_3CH_2$ | $(CH_2)_2$ | $(CH_2)_2$ | $b^2$ | 39 | 110-145 (HCl) |
| 9 | " | $CH_3$ | $CH_3$ | 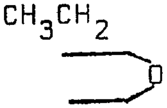 | | $(CH_2)_2$ | $(CH_2)_2$ | $b^2$ | 53 | 63 (base) |
| 10 | " | $CH_3$ | $CH_3$ | $(CH_3)_2CH$ | $PhCH_2$ | $(CH_2)_2$ | $(CH_2)_2$ | $b^2$ | 75 | 110 (HCl) |
| 11 | " | $CH_3$ | $CH_3$ | $CH_3$ | $CH_3$ | $(CH_2)_2$ | $(CH_2)_3$ | $a^2$ | 35 | oil |
| 12 | " | $CH_3$ | $CH_3OCH_2CH_2$ | $(CH_3)_2CH$ | $PhCH_2$ | $(CH_2)_2$ | $(CH_2)_2$ | $b^2$ | 53 | oil |
| 13 | " | $CH_3$ | $CH_3OCH_2CH_2$ | 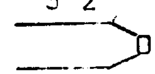 | | $(CH_2)_2$ | $(CH_2)_2$ | $a^2$ | 61 | oil |
| 14 | " | $CH_3$ | $CH_3$ | $CH_3$ | $CH_3$ | $(CH_2)_2$ | $CH(CH_3)CH_2$ | $b^2$ | 8 | oil |

$^1H$-NMR and $^{13}C$-NMR have been obtained for all compounds present in Table I.

Example 3 (new process)

Preparation of 2,6-dimethyl-4-(2,3-dichlorophenyl)-1,4-dihydropyridine-3,5-dicarboxylic acid 3-ethyl-5-methyl ester

1.95 g of 2,6-dimethyl-4-(2,3-dichlorophenyl)-1,4-dihydropyridine-3,5-dicarboxylic acid-3-[2-(2-N,N-dimethylaminoethylthio) ethyl]-5-methyl ester was dissolved in 50 ml of ethanol. Raney nickel was added and the mixture was refluxed for 10 minutes. The mixture was then filtered and evaporated. To the residue was then added 50 ml of 1 M HCl and the mixture was extracted with ether. The ether phase was dried and evaporated. The residue was chrystallized from isopropylether. The title compound was obtained; melting point: 145°C. $^1H$-NMR spectrum was consistent with the given structure. Yield: 73 %.

Example 4

The following examples illustrates the preparation of pharmaceutical compositions of the invention. The wording "active substance" denotes a compound according to the present invention or a salt thereof and preferably the compound according to Example 1.

Formulation A

A syrup containing 2% (weight per volume) of active substance was prepared from the following ingredients:

| | | |
|---|---|---|
| Active substance | | 2.0 g |
| Saccharine | | 0.6 g |
| Sugar | | 30.0 g |
| Glycerine | | 5.0 g |
| Flavouring agent | | 0.1 g |
| Ethanol 96% | | 10.0 g |
| Distilled water | ad | 100.0 g |

Sugar, saccharine and the active substance were dissolved in 60 g of warm water. After cooling, glycerine and solution of flavouring agents dissolved in ethanol were added. To the mixture water was then added to 100 ml.

Formulation B

Active substance (250 g) was mixed with lactose (175.8 g), potato starch (169.7 g) and colloidal silicic acid (32 g). The mixture was moistened with a 10% of gelatine and was granulated through a 12-mesh sieve. After drying potato starch (160 g), talc (50 g) and magnesium stearate (5 g) were admixed and the mixture thus obtained was pressed into tablets (10.000), each containing 25 mg of active substance. The tablets are provided with a breaking score to give another dose than 25 mg or to give multiples thereof when broken.

Formulation C

Granules were prepared from active substance (250 g), lactose (175.9 g) and an alcoholic solution of polyvinylpyrrolidone (25 g). After the drying step the granules were mixed with talc (25 g), potato starch (40 g) and magnesium stearate (2.50 g) and were pressed into 10.000 tablets being biconvex. These tablets are coated with a 10% alcoholic solution of shellac and thereupon with an aqueous solution containing saccharose (45%), gum arabicum (5%), gelatine (4%) and dyestuff (0.2%). After the first five coatings talc and

powdered sugar were used for powdering. The priming coat was then coated with a 66% sugar syrup and polished with a 10% carnauba wax solution in carbon tetrachloride.

BIOLOGICAL TESTS

The antihypertensive effect of the compounds was tested in conscious, unrestrained spontaneously hypertensive rats (SHR) of the Okamoto strain. The animals had been prepared by prior implantation of indwelling catheters in the abdominal aorta via the femoral artery. Mean arterial blood pressure (MABP) and heart rate were continuously monitored. After a 2 hour control period the compound under study was administered by oral intubation at 2 hour intervals, suspended in methocel solution (5 ml/kg bodyweight). The cumulated doses were 1, 5 and 25 µmoles/kg bodyweight. The antihypertensive response, i.e. the BP reduction to each dose, was expressed as a percentage of the initial control BP level and plotted against the dose on a logarithmic scale. The dose which would give 20 per cent BP reduction was then determined by interpolation. The results are shown in table 2.

The specificity towards smooth muscle relaxation was examined as follows: The isolated portal vein preparation of Wistar rats was mounted in an organ bath together with a paced isolated papillary heart muscle preparation of the same animal. The integrated contractile activity of the portal vein smooth muscle and the peak force amplitude of the papillary, myocardial, preparation were recorded. The respective activities during a 30 min control period were set as 100 per cent and the ensuing activities under the influence of an agent under study were expressed as a percentage thereof. The agent was administered at 10 min intervals and the potency for vasodilatation (-log $ED_{50}$ of portal vein) and that of myocardial depression (-log $ED_{50}$ of papillary muscle) were determined by interpolation from the concentration-effect relationships determined in each

experiment. A "separation" value was determined for each compound by averaging the differences of the -log $ED_{50}$ values for vasodilatation and myocardial depression, respectively, ogtained in the experiments. This logarithmic separation value was transformed into numeric format and entered into table 2.

The compounds of the invention were compared with Nifedipin [2,6-dimethyl-4-(2-nitrophenyl)-1,4-dihydropyridine-3,5-dicarboxylic acid-3,5-dimethylester].

Table 2

| Compound no according to Table I | SHR $ED_{20}$ μmoles/kg bodyweight | Ratio heart vasc. |
|---|---|---|
| Nifedipine | 5 | 15 |
| 1 | 16 | 30 |
| 2 | 30 | 5 |
| 3 | 50 | 18 |
| 4 | 19 | 3 |
| 5 | 20 | 19 |
| 6 | 25 | 16 |
| 7 | 56 | 20 |
| 8 | 6 | 4 |
| 9 | 5 | 27 |
| 10 | 9 | 22 |
| 11 | | 3 |
| 12 | 26 | 50 |
| 13 | 15 | 70 |
| 14 | | |

Claims

1. A compound of the formula

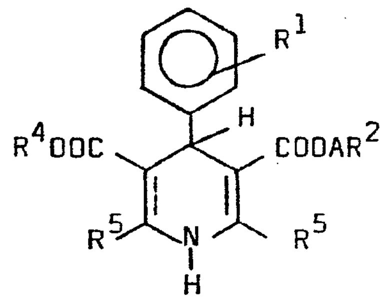

I

wherein $R^1$ is one or two (the same or different) substituents on the phenyl ring selected from the group consisting of lower alkyl, $NO_2$, CN, $CF_3$, Cl and Br; A is a straight or branched alkylene group with 2-5 carbon atomms; $R^2$ is

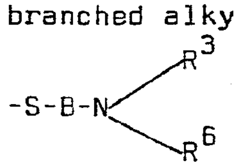

wherein B is a straight or branched alkylene group with 2-5 carbon atoms and $R^3$ and $R^6$ are the same or different and each represents a straight or branched alkyl group with 1-5 carbon atoms, an alkylaryl group or both together are part of a 5-7 membered heterocyclic ring; $R^4$ is a straight or branched alkyl group with 1-5 carbon atoms or a straight or branched alkyl·group with 1-5 carbon atoms in which alkyl group the carbon chain is interrupted by an oxygen atom; $R^5$ is a lower alkyl group, or a physiologically acceptable salt or optical isomer thereof.

2. A compound according to claim 1;

1) 2,6-dimethyl-4-(2,3-dichlorophenyl)-1,4-dihydropyridine-3,5-dicarboxylic acid-3-[2-(2-N,N-dimethylaminoethylthio)-ethyl]-5-methyl ester

2) 2,6-dimethyl-4-(2,3-dichlorophenyl)-1,4-dihydropyridine-3,5-dicarboxylic acid-3-[2-(2-N,N-dimethylaminoethylthio)-ethyl]-5-(2-methylethyl)ester

3) 2,6-dimethyl-4-(2,3-dichlorophenyl)-1,4-dihydropyridine-3,5-dicarboxylic acid-3-[2-(2-N,N-dimethylaminoethylthio)-ethyl]-5-ethyl ester

4) 2,6-dimethyl-4-(2,3-dichlorophenyl)-1,4-dihydropyridine-3,5-dicarboxylic acid-3-[2-(2-N,N-dimethylaminoetylthio-ethyl]-5-(2-methoxyethyl) ester

5) 2,6-dimethyl-4-(2,3-dichlorophenyl)-1,4-dihydropyridine-3,5-dicarboxylic acid-3-[2-(2-N,N-diethylaminoethylthio)-ethyl]-5-methyl ester

6) 2,6-dimethyl-4-(2,3-dichlorophenyl)-1,4-dihydropyridine-3,5-dicarboxylic acid-3-[2-(2-morpholinoethylthio)-ethyl]-5-methyl ester

7) 2,6-dimethyl-4-(2,3-dichlorophenyl)-1,4-dihydropyridine-3,5-dicarboxylic acid-3-[2-(N-isopropyl-N-benzylaminoethylthio)ethyl]-5-methyl ester

8) 2,6-dimethyl-4-(2,3-dichlorophenyl)-1,4-dihydropyridine-3,5-dicarboxylic acid-3-[2-(3-N,N-dimethylaminopropylthio)-ethyl]-5-methyl ester

9) 2,6-dimethyl-4-(2,3-dichlorophenyl)-1,4-dihydropyridine-3,5-dicarboxylic acid-3-[2-(2-N-isopropyl-N-benzylethylthio)ethyl]-5-(2-methoxyethyl) ester

10) 2,6-dimethyl-4-(2,3-dichlorophenyl)-1,4-dihydropyridine-3,5-dicarboxylic acid-3-[2-(2-morpholinoethylthio)-ethyl]-5-(2-methoxyethyl) ester

11) 2,6-dimethyl-4-(2-nitrophenyl)-1,4-dihydropyridine-3,5-dicarboxylic acid-3-[2-(2-N,N-dimethylaminoethylthio)ethyl]-5-methyl ester

12) 2,6-dimethyl-4-(3-nitrophenyl)-1,4-dihydropyridine-3,5-dicarboxylic acid-3-[2-(N,N-dimethylaminoethylthio)ethyl]-5-methyl ester

13) 2,6-dimethyl-4-(3-cyanophenyl)-1,4-dihydropyridine-3,5-dicarboxylic acid-3-[2-(2-N,N-dimethylaminoethylthio) ethyl]-5-methyl ester

3. A compound according to any of claims 1-2 in the form of an optical isomer thereof.

4. A compound according to any of claims 1-3 in the form of a physiologically acceptable salt thereof.

5. A process for preparing a compound of the formula

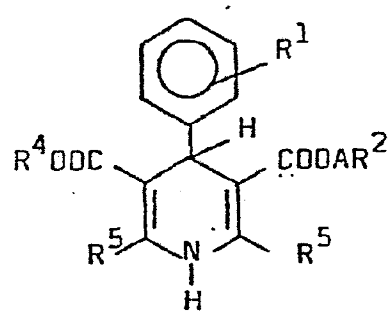

I

wherein $R^1$ is one or two (the same or different) substituents on the phenyl ring selected from the group consisting of lower alkyl, $NO_2$, CN, $CF_3$, Cl and Br; A is a straight or branched alkylene group with 2-5 carbon atomms; $R^2$ is

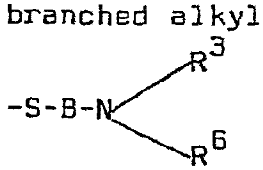

wherein B is a straight or branched alkylene group with 2-5 carbon atoms and $R^3$ and $R^6$ are the same or different and each represents a straight or branched alkyl group with 1-5 carbon atoms, an alkylaryl group or both together are part of a 5-7 membered heterocyclic ring; $R^4$ is a straight or branched alkyl group with 1-5 carbon atoms or a straight or branched alkyl group with 1-5 carbon atoms in which alkyl group the carbon chain is interrupted by an oxygen atom; $R^5$ is a lower alkyl group, or a physiologically acceptable salt or optical isomer thereof.

characterized in that

a[1]) a compound of the formula

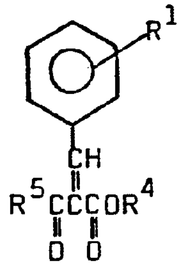

II a

wherein $R^1$, $R^4$ and $R^5$ have the meanings given above, is reacted with a compound of the formula

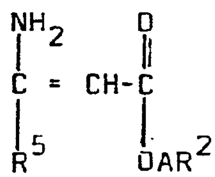

III a

wherein A and $R^2$ and $R^5$ have the meanings given above, to the formation of a compound of the formula I; or

a[2]) a compound of the formula

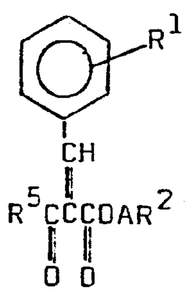

II b

wherein $R^1$, $R^2$, $R^5$ and A have the meanings given above, is reacted with a compound of the formula

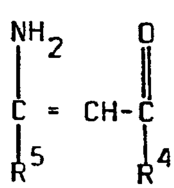

III b

wherein $R^4$ and $R^5$ have the meaning given above, to the formation of a compound of the formula I; or

$b^1$) a compound of the formula

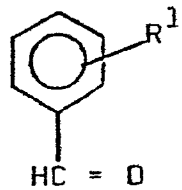

IV

wherein $R^1$ has the meaning given above, is reacted with compounds of the formulas

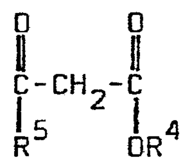

V a

and

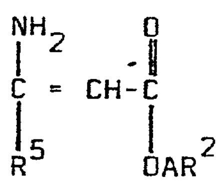

III a

wherein $R^2$, $R^4$, $R^5$ and A have the meanings given above, to the formation of a compound of the formula I; or

$b^2$) a compound of formula IV above, wherein $R^1$ has the meaning given above, is reacted with compounds of the formulas

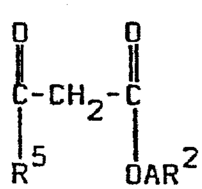

V b

and

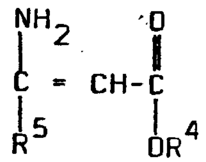

III b

wherein $R^2$, $R^4$, $R^5$ and A have the meanings given above, to the formation of a compound of the formula I; or

$c^1$) a compound of formula II a above, wherein $R^1$, $R^4$ and $R^5$ have the meanings given above, is reacted with a compound of the formula

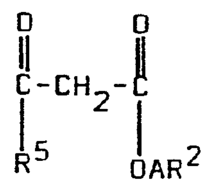

VI a

wherein $R^2$, $R^5$ and A have the meanings given above, in the presence of ammonia, to the formation of a compound of the formula I; or

$c^2$) a compound of formula II b above, wherein $R^1$, $R^2$, $R^5$ and A have tne meanings given above, is reacted with a compound of the formula

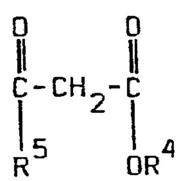

VI b

wherein $R^4$ and $R^5$ have the meanings given above, in the presence of ammonia, to the formation of a compound of the formula I, or

d) a compound of formula IV above, wherein $R^1$ has the meaning given above, is reacted with the compounds of the formulas V a and V b, wherein $R^2$, $R^4$, $R^5$ and A have the meanings given above, in the presence of ammonia, to the formation of a compound of the formula I, whereafter, if desired, the compound obtained by any of methods $a^1$) - d) is converted to a physiologically acceptable salt thereof and/or converted to a substantially pure stereoisomer thereof.

6. A process according to claim 5 characterized in that a compound according to any of claims 1-4 is prepared.

7. A pharmaceutical preparation comprising as active ingredient a compound according to any of claims 1-4 or a physiologically acceptable salt or an optical isomer thereof.

8. A pharmaceutical preparation according to claim 7 in dosage unit form.

9. A pharmaceutical preparation according to claims 7-8 comprising the active ingredient in association with a pharmaceutically acceptable carrier.

10. The use of a compound according to any of claims 1-4 or a physiologically acceptable salt thereof for the preparation of a pharmaceutical preparation comprising as an active ingredient an amount of said compound.

11. Use of a compound according to any of claims 1-4 as a drug for the treatment of cardiovascular diseases and other disorders where smooth muscle relaxation is therapeutically important, particularly hypertension.

12. The use of a compound according to any of claims 1-2 or a salt or an optical isomer thereof as an intermediate for the preparation of therapeutically active dihydropyridines.

13. A process for preparing a compound of the formula

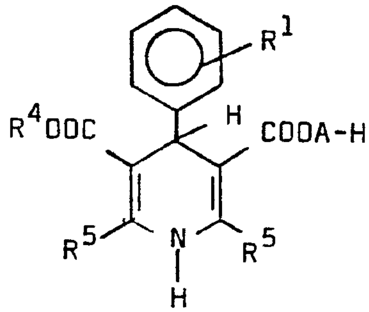

I A

wherein A is a straight or branched alkylene group with 2-5 carbon atoms and $R^1$ is one or two (the same or different) substituents on the phenyl ring selected from the group consisting of lower alkyl, $NO_2$, CN, $CF_3$, Cl or Br; $R^4$ is a straight or branched alkyl group with 1-5 carbon atoms or a straight or branched alkyl group with 1-5 carbon atoms in which alkyl group the carbon chain is interrupted by an oxygen atom; $R^5$ is a lower alkyl group; or a physiologically acceptable salt or optical isomer thereof, characterized in that a compound of the formula

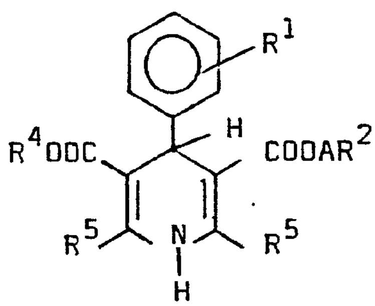

I

wherein $R^1$ is one or two (the same or different) substituents on the phenyl ring selected from the group consisting of lower alkyl, $NO_2$, CN, $CF_3$, Cl and Br; A is a straight or branched alkylene group with 2-5 carbon atoms; $R^2$ is

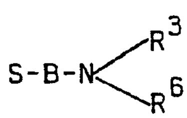

wherein B is a straight or branched alkylene

group with 2-5 carbon atoms and $R^3$ and $R^6$ are the same or different and each represents a straight or branched alkyl group with 1-5 carbon atoms, an alkylaryl group or both together are part of a 5-7 membered heterocyclic ring; $R^4$ is a straight or branched alkyl group with 1-5 carbon atoms or a straight or branched alkyl group with 1-5 carbon atoms in which alkyl group the carbon chain is interrupted by an oxygen atom; $R^5$ is a lower alkyl group, or a physiologically acceptable salt or optical isomer thereof, is treated in a solvent with a Raney catalyst.

Claims for Austria

1. A process for preparing a compound of the formula

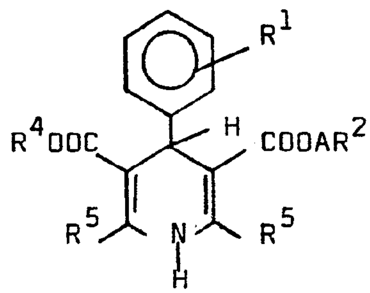

I

wherein $R^1$ is one or two (the same or different) substituents on the phenyl ring selected from the group consisting of lower alkyl, $NO_2$, CN, $CF_3$, Cl and Br; A is a straight or branched alkylene group with 2-5 carbon atoms; $R^2$ is

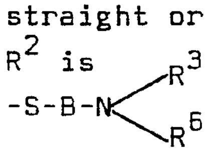

wherein B is a straight or branched alkylene group with 2-5 carbon atoms and $R^3$ and $R^6$ are the same or different and each represents a straight or branched alkyl group with 1-5 carbon atoms, an alkylaryl group or both together are part of a 5-7 membered heterocyclic ring; $R^4$ is a straight or branched alkyl group with 1-5 carbon atoms or a straight or branched alkyl group with 1-5 carbon atoms in which alkyl group the carbon chain is interrupted by an oxygen atom; $R^5$ is a lower alkyl group; or a physiologically acceptable salt or optical isomer thereof, characterized in that

$a^1$) a compound of the formula

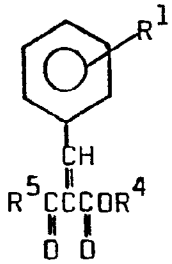

II a

wherein $R^1$, $R^4$ and $R^5$ have the meanings given above, is reacted with a compound of the formula

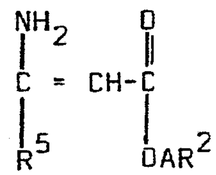

III a

wherein A and $R^2$ and $R^5$ have the meanings given above, to the formation of a compound of the formula I; or

$a^2$) a compound of the formula

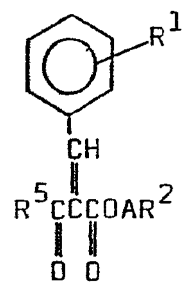

II b

wherein $R^1$, $R^2$, $R^5$ and A have the meanings given above, is reacted with a compound of the formula

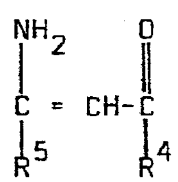

III b

wherein $R^4$ and $R^5$ have the meaning given above, to the formation of a compound of the formula I; or

$b^1$) a compound of the formula

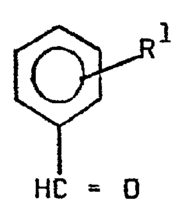

IV

wherein $R^1$ has the meaning given above, is reacted with compounds of the formulas

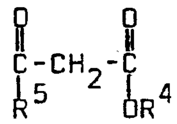

V a

and

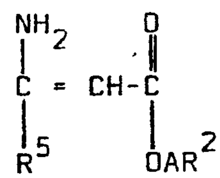

III a

wherein $R^2$, $R^4$, $R^5$ and A have the meanings given above, to the formation of a compound of the formula I; or

$b^2$) a compound of formula IV above, wherein $R^1$ has the meaning given above, is reacted with compounds of the formulas

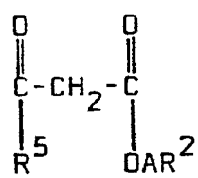

V b

and

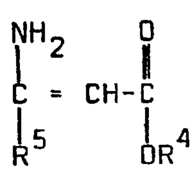

III b

wherein $R^2$, $R^4$, $R^5$ and A have the meanings given above, to the formation of a compound of the formula I; or

$c^1$) a compound of formula II a above, wherein $R^1$, $R^4$ and $R^5$ have the meanings given above, is reacted with a compound of the formula

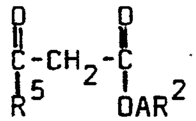

VI a

wherein $R^2$ and $R^5$ and A have the meanings given above, in the presence of ammonia, to the formation of a compound of the formula I; or

$c^2$) a compound of formula II b above, wherein $R^1$, $R^2$, $R^5$ and A have the meanings given above, is reacted with a compound of the formula

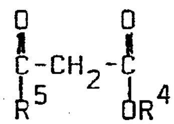

VI b

wherein $R^4$ and $R^5$ have the meanings given above, in the presence of ammonia, to the formation of a compound of the formula I, or

d) a compound of formula IV above, wherein $R^1$ has the meaning given above, is reacted with the compounds of the formulas V a and V b, wherein $R^2$, $R^4$, $R^5$ and A have the meanings given above, in the presence of ammonia, to the formation of a compound of the formula I, whereafter, if desired, the compound obtained by any of methods $a^1$) - d) is converted to a physiologically acceptable salt thereof and/or converted to a substantially pure stereoisomer thereof.

2. A process according to claim 1, characterized in preparing the compound

1) 2,6-dimethyl-4-(2,3-dichlorophenyl)-1,4-dihydropyridine-3,5-dicarboxylic acid-3-[2-(2-N,N-dimethylaminoethylthio)-ethyl]-5-methyl ester, or

2) 2,6-dimethyl-4-(2,3-dichlorophenyl)-1,4-dihydropyridine-3,5-dicarboxylic acid-3-[2-(2,N,N-dimethylaminoethylthio)-ethyl]-5-(2-methylethyl)ester, or

3) 2,6-dimethyl-4-(2,3-dichlorophenyl)-1,4-dihydropyridine-3,5-dicarboxylic acid-3-[2-(2-N,N-dimethylaminoethylthio)-ethyl]-5-ethyl ester, or

4) 2,6-dimethyl-4-(2,3-dichlorophenyl)-1,4-dihydropyridine-3,5-dicarboxylic acid-3-[2-(2-N,N-dimethylaminoetylthio-ethyl]-5-(2-methoxyethyl) ester, or

5) 2,6-dimethyl-4-(2,3-dichlorophenyl)-1,4-dihydropyridine-3,5-dicarboxylic acid-3-[2-(2-N,N-diethylaminoethylthio)-ethyl]-5-methyl ester, or

6) 2,6-dimethyl-4-(2,3-dichlorophenyl)-1,4-dihydropyridine-3,5-dicarboxylic acid-3-[2-(2-morpholinoethylthio)-ethyl]-5-methyl ester , or

7) 2,6-dimethyl-4-(2,3-dichlorophenyl)-1,4-dihydropyridine-3,5-dicarboxylic acid-3-[2-(N-isopropyl-N-benzylaminoethylthio)ethyl]-5-methyl ester, or

8) 2,6-dimethyl-4-(2,3-dichlorophenyl)-1,4-dihydropyridine-3,5-dicarboxylic acid-3-[2-(3-N,N-dimethylaminopropylthio)-ethyl]-5-methyl ester, or

9) 2,6-dimethyl-4-(2,3-dichlorophenyl)-1,4-dihydropyridine-3,5-dicarboxylic acid-3-[2-(2-N-isopropyl-N-benzylethylthio)ethyl]-5-(2-methoxyethyl) ester , or

10) 2,6-dimethyl-4-(2,3-dichlorophenyl)-1,4-dihydropyridine-3,5-dicarboxylic acid-3-[2-(2-morpholinoethylthio)-ethyl]-5-(2-metnoxyethyl) ester, or

11) 2,6-dimethyl-4-(2-nitrophenyl)-1,4-dihydropyridine-3,5-dicarboxylic acid-3-[2-(2-N,N-dimethylaminoethylthio)ethyl]-5-methyl ester, or

12) 2,6-dimethyl-4-(3-nitrophenyl)-1,4-dihydropyridines--3.5-dicarboxylic acid-3-[2-(N,N-dimethylaminoethylthio)-ethyl]-5-methyl ester, or

13) 2,6-dimethyl-4-(3-cyanophenyl)-1,4-dihydropyridine--3,5-dicarboxylic acid-3-[2-(2-N,Npdimethylaminoethylthio)-ethyl]-5-methyl ester or a physiologically acceptable salt or optical isomer thereof.

3. A process according to claim 1 or 2 characterized in preparing a compound in the form of an optical isomer thereof.

4. A process according to claim 1-3 characterized in preparing a compound in the form of a physiologically acceptable salt thereof.

5. The use of a compound according to any of claims 1-4 or a salt or an optical isomer thereof as an intermediate for the preparation of therapeutically active dihydropyridines.

6. A process for preparing a compound of the formula

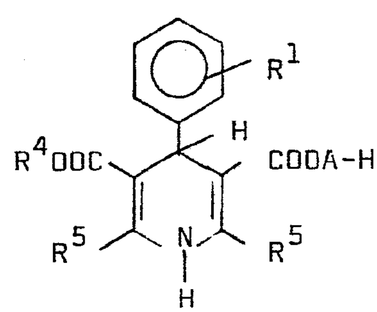

I A

wherein A is a straight or branched alkylene group with 2-5 carbon atoms and $R^1$ is one or two (the same or different) substituents on the phenyl ring selected from the group consisting of lower alkyl, $NO_2$, CN, $CF_3$, Cl or Br; $R^4$ is a straight or branched alkyl group with 1-5 carbon atoms or a straight or branched alkyl group with 1-5 carbon atoms

in which alkyl group the carbon chain is interrupted by an oxygen atom; $R^5$ is a lower alkyl group; or a physiologically acceptable salt or optical isomer thereof, characterized in that a compound of the formula

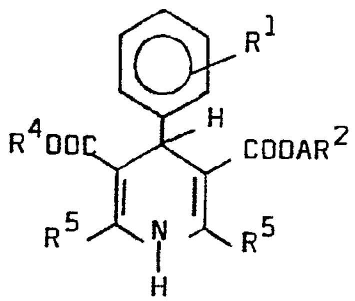

I

wherein $R^1$ is one or two (the same or different) substituents on the phenyl ring selected from the group consisting of lower alkyl, $NO_2$, CN, $CF_3$, Cl and Br; A is a straight or branched alkylene group with 2-5 carbon atoms; $R^2$ is

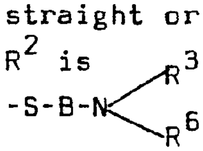

wherein B is a straight or branched alkylene group with 2-5 carbon atoms and $R^3$ and $R^6$ are the same or different and each represents a straight or branched alkyl group with 1-5 carbon atoms, an alkylaryl group or both together are part of a 5-7 membered heterocyclic ring; $R^4$ is a straight or branched alkyl group with 1-5 carbon atoms or a straight or branched alkyl group with 1-5 carbon atoms in which alkyl group the carbon chain is interrupted by an oxygen atom; $R^5$ is a lower alkyl group, or a physiologically acceptable salt or optical isomer thereof, is treated in a solvent with a Raney catalyst.